# FASCICULE DE BREVET EUROPEEN

(11) **EP 3 315 169 B1**
(45) Date de publication et mention de la délivrance du brevet: **13.11.2019**
(21) Numéro de dépôt: 16195548.9
(22) Date de dépôt: 25.10.2016
(51) Int. Cl.: A61N 5/06

(54) **SUPPORT DE MODULES D'IRRADIATION TRANSCUTANÉE ET DISPOSITIF ASSOCIÉ**
HALTERUNG FÜR MODULE ZUR TRANSKUTANEN BESTRAHLUNG, UND ENTSPRECHENDE VORRICHTUNG
MOUNTING FOR TRANSCUTANEOUS IRRADIATION MODULES AND ASSOCIATED DEVICE

(43) Date de publication de la demande: 02.05.2018
(73) Titulaire: REGENLIFE, 34000 Montpellier (FR)
(72) Inventeur: BLIVET, Guillaume, 34070 Montpellier (FR); COCHARD, Etienne, 34670 Baillargues (FR); MOREAU, Guillaume, 34670 Baillargues (FR)
(74) Mandataire: Regimbeau

(56) Documents cités:
- US-A1- 2010 204 762

## Description

L'invention s'inscrit dans le domaine du traitement par irradiation transcutanée.

L'invention porte plus particulièrement sur un dispositif permettant la mise en œuvre d'un traitement par irradiation transcutanée.

L'irradiation transcutanée est une technique connue par laquelle un rayonnement d'ondes ou de particules est émis au contact de la peau et pénètre en profondeur.

Parmi ces techniques, on connait notamment la photobiomodulation et la thérapie laser de basse intensité (LLLT : Low Level Laser Therapy) utilisant des diodes lasers et/ou des diodes électroluminescentes (LEDs) et permettant de réparer et régénérer des tissus endommagés. Cette technique consiste à positionner une sonde sur la peau d'un patient et à réaliser l'émission photonique pendant un temps donné au niveau de la zone tissulaire endommagée. La sonde, par exemple commercialisée par la société THOR, comporte une tête d'émission placée sur la peau, une poignée de maintien de la tête d'émission et des câbles d'alimentation reliés à une unité de contrôle. La tête d'émission est maintenue en place par le praticien pendant toute la séance.

Ces techniques d'irradiations transcutanées, notamment par photothérapie, s'appliquent également aux traitements neurologiques et psychiatriques. On parle alors d'irradiation transcrânienne. Dans ce cas, une sonde d'émission lumineuse allant du visible à l'infrarouge, du type de celle précédemment décrite, est positionnée et maintenue à la surface de la tête du patient par le praticien. Il est possible d'agir par cette technique sur des troubles neurologiques de façon thérapeutique pour restaurer les facultés neurologiques, stopper la progression des troubles cognitifs, par exemple touchant les maladies neurodégénératives de type Alzheimer, ou maintenir une qualité de vie.

L'application de ces techniques aux traitements neurologiques nécessite un positionnement précis de la source lumineuse dirigée vers une partie du cerveau impliquée dans le trouble à soigner. Il s'agit naturellement de viser uniquement la zone concernée, au risque d'affecter une zone voisine. Pour ce faire, un dispositif connu et commercialisé par la société NEUROTHERA prévoit un support de sonde formant casque et comportant des anneaux répartis sur la tête du patient. Le casque est maintenu en position au moyen d'une sangle comportant une mentonnière qui s'attache de chaque côté à deux anneaux périphériques. Chaque anneau est un indicateur de position vers une zone cible du cerveau. La sonde est positionnée par le praticien au niveau d'un anneau et maintenue dans cette position pendant la durée du traitement.

Ce dispositif est néanmoins insuffisant pour assurer un positionnement précis de l'émission lumineuse vers une zone cible. En effet, le maintien de la sonde par le praticien peut engendrer un changement d'angle de l'émission lumineuse même lorsque la sonde reste en position dans l'anneau. En outre, le praticien doit nécessairement maintenir la sonde pendant le traitement. Il n'est d'ailleurs pas possible, dans ce système, d'effectuer des traitements simultanés de plusieurs zones du cerveau au regard du fait que chaque sonde est maintenue en place par le praticien et qu'un dispositif de contrôle encombrant est en liaison avec la sonde. Pourtant, l'efficacité de certains traitements neurologiques peut être conditionnée par la possibilité de pouvoir réaliser des irradiations sur plusieurs zones en même temps pour stimuler de façon concomitante des parties ciblées du cerveau.

Le document US 2010 204 762 décrit un support de module d'irradiation transcutanée, comportant une pluralité d'anneaux reliés entre eux par des éléments de jonction en formant un support de module d'irradiation transcrânienne apte à être positionné sur la tête d'un utilisateur,.

L'invention vise un dispositif permettant de réaliser l'irradiation transcutanée d'une ou de plusieurs zones précises pour des traitements neurologiques et psychiatriques, voire d'autres types.

L'invention vise également un dispositif permettant d'assurer l'irradiation simultanée de plusieurs zones ciblées.

L'invention s'applique aux traitements neurologiques, psychiatriques, ou autres. Dans le cas de l'application aux traitements neurologiques et psychiatriques, l'invention vise un système assurant un positionnement adapté à la morphologie de la tête du patient de la ou des sources d'irradiations.

À cet effet, l'invention porte en premier lieu sur un support de modules d'irradiation transcutanée qui est essentiellement caractérisé en ce qu'il comporte des moyens de positionnement dudit support au niveau de la zone à irradier, et au moins un anneau réalisé en un matériau élastique et/ou souple et apte à assurer la fixation par emprise élastique d'un module d'irradiation.

Le support de l'invention comporte aussi une pluralité d'anneaux reliés entre eux par des éléments de jonction en formant un support de module d'irradiation transcrânienne apte à être positionné sur la tête d'un utilisateur et les anneaux comportent des anneaux périphériques dont certains au moins ne sont pas reliés entre eux par des éléments de jonction ou les anneaux périphériques ne sont pas reliés entre eux par des éléments de jonction.

Le support peut également comporter les caractéristiques optionnelles suivantes considérées isolément ou selon toutes les combinaisons techniques possibles :
- les anneaux sont symétriquement disposés de part et d'autre d'un axe coïncidant avec l'axe médian de la tête lorsque le support est en place sur la tête de l'utilisateur.
- les anneaux comportent des anneaux de seconde périphérie et quatre anneaux centraux, au moins certains anneaux de seconde périphérie ne sont pas reliés entre eux par des éléments de jonction, et les quatre anneaux centraux sont reliés entre eux par des éléments de jonction.
- le support comporte une sangle à serrage contrôlé comportant au moins une partie de liaison reliant les anneaux périphériques.
- la sangle comporte une mentonnière se prolongeant de chaque côté du support jusqu'à deux éléments de liaisons qui sont destinés à être respectivement disposés de chaque côté d'une oreille de l'utilisateur et qui forment jonction entre ladite mentonnière et ladite partie de liaison.
- la face interne de chaque anneau comporte une rainure d'aide à la fixation du module dédié.
- les anneaux sont réalisés en un matériau élastique souple et/ou élastique.

L'invention porte également sur un dispositif d'irradiation transcutanée qui comporte un support tel que précédemment défini et au moins un module d'irradiation comportant au moins une source de rayonnement et qui est apte à être coaxialement fixé sur ledit anneau.

Avantageusement, la source de rayonnement comporte une série de diodes laser et/ ou de diodes électroluminescentes (LEDs) émettant dans les spectres visibles ou infrarouge.

D'autres caractéristiques et avantages de l'invention ressortiront clairement de la description qui en est donnée ci-dessous, à titre indicatif et nullement limitatif, en référence aux figures annexées parmi lesquelles :
- la figure 1 est une vue schématique de dessus du support de modules d'irradiation transcutanée de l'invention,
- la figure 2 est une vue schématique de côté du support de modules d'irradiation transcutanée de l'invention,
- la figure 3 est une vue schématique de dessus du support de modules d'irradiation transcrânienne transcutanée de l'invention en position sur la tête d'un patient,
- la figure 4 est une vue schématique de côté du support de modules d'irradiation transcrânienne de l'invention en position sur la tête d'un patient,
- la figure 5 est une vue schématique de face du support de modules d'irradiation transcrânienne de l'invention en position sur la tête d'un patient,
- la figure 6 est une vue schématique de dos du support de modules d'irradiation transcrânienne de l'invention en position sur la tête d'un patient,
- la figure 7 est une vue schématique de côté du support de modules d'irradiation transcrânienne de l'invention en position sur la tête d'un patient sur laquelle est également illustrée la sangle de maintien dudit support,
- la figure 8 est une vue en coupe selon la ligne VIII-VIII de la figure 7 d'un anneau du support de l'invention muni d'une rainure interne,
- la figure 9 est une vue générale en perspective de devant du dispositif de l'invention comportant le support de modules d'irradiation et quatre modules en position dans quatre anneaux situés au niveau du lobe frontal, et
- la figure 10 est une vue en coupe selon la ligne X-X de la figure 9 d'un module en position dans un anneau du support de l'invention en position sur la peau de la tête d'un patient.

L'invention est définie dans les revendications, d'autres modes de réalisation étant simplement des exemples.

Le support de modules de l'invention est essentiellement caractérisé en ce qu'il comporte un ou des anneaux réalisés dans un matériau souple et/ou élastique permettant d'assurer par emprise élastique le maintien fixe d'un module d'irradiation transcutanée, dans cet exemple d'irradiation transcrânienne. On entend par matériau souple et/ou élastique un matériau par exemple élastomère ou en caoutchouc qui autorise l'insertion d'un module de surface externe essentiellement cylindrique dans l'anneau par écartement dudit anneau et retour élastique contre le module. Le matériau pourra être souple sans être élastique, son écartement pour l'insertion et la fixation du module impliquant une sensible extension du matériau, le maintien du module dans l'anneau pouvant alors être assuré par friction. Le support comporte également des moyens de positionnement sur la zone à irradier. Ces moyens peuvent prendre la forme d'une sangle, mais peuvent également, ou en complément, tenir à la forme du support qui, en reposant sur la zone à traiter, s'adaptent à cette zone et sont maintenus en place du fait de cette forme de support adapté.

On entend par irradiation, l'exposition volontaire à des rayonnements propagatoires d'énergie par des ondes ou des particules. Les rayonnements qui intéressent plus particulièrement le cadre de l'invention sont les rayonnements électromagnétiques allant du spectre visible à l'infrarouge. L'invention pourra également s'appliquer à l'émission de champs électromagnétiques, de champs électriques, de vibrations sonores ou à une combinaison de ces différents types d'émissions.

Le support de module de l'invention est plus particulièrement appliqué aux traitements neurologiques, par exemple les pathologies neurodégénératives de type Alzheimer, et nécessite ainsi d'être apposé à la surface de la tête d'un patient. La configuration du support doit ainsi s'adapter à la forme de la tête.

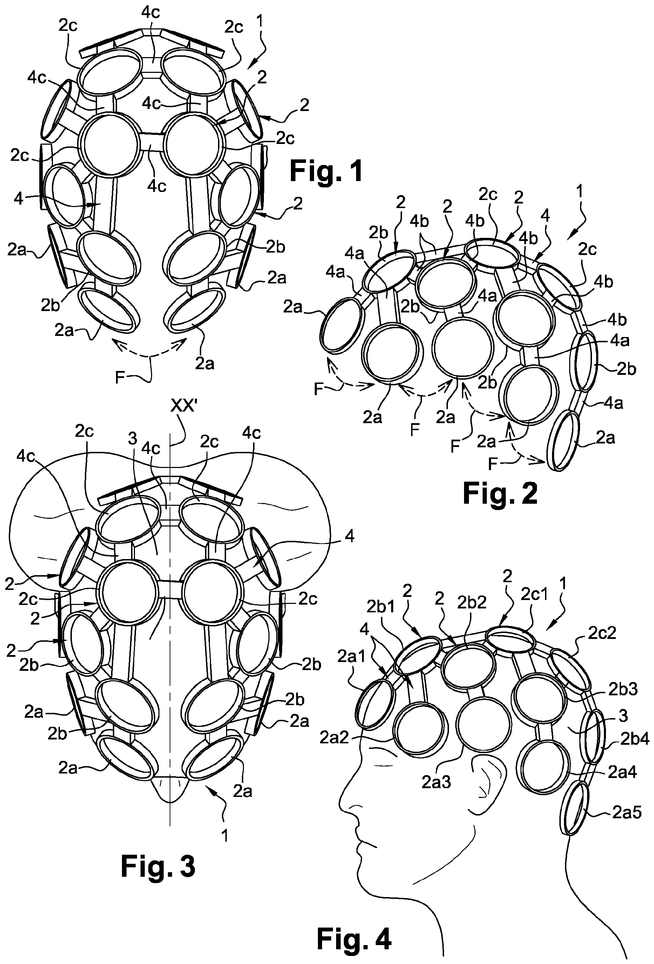

On se réfère aux figures 1 à 6 pour décrire le support de modules de l'invention appliqué aux traitements neurologiques.

Le support 1 comporte une pluralité d'anneaux 2 de maintien de modules répartis à la surface de la tête 3 (pour des raisons de clarté, tous les anneaux ne sont pas référencés sur les figures). À titre d'exemple non limitatif, chaque anneau est réalisé en silicone, présente un rayon externe de 25 millimètres, un rayon interne de 23 millimètres (soit une épaisseur de 2 millimètres), et une hauteur de 6 millimètres. La hauteur doit être suffisante à la tenue d'un module qui sera décrit plus loin. En variante de l'utilisation de silicone, les anneaux peuvent être réalisés en caoutchouc, en matériau élastomère ou en tout autre matériau polymérique ou non qui est suffisamment souple pour permettent l'insertion et le maintien d'un module de surface externe essentiellement cylindrique.

Les anneaux 2 sont répartis symétriquement sur le support, de façon que lorsqu'ils sont positionnées sur la tête 3, l'axe de symétrie du support XX' coïncide avec l'axe médian XX' de la tête 3 de l'utilisateur. Les anneaux 2 sont reliés en entre eux par des éléments de jonction souples 4, réalisés par exemple dans le même matériau que les anneaux, dans cet exemple en silicone. Les éléments de jonction 4 sont précisément positionnés sur le support 1 pour permettre à la fois, au support 1 de s'adapter à la forme de la tête 3 de l'utilisateur, et pour permettre le maintien du support sur la tête 3, comme il sera décrit en détail plus loin.

Les anneaux 2 se répartissent de la façon suivante : le support 1 prévoit dix anneaux périphériques 2a, quatre anneaux de seconde périphérie 2b et quatre anneaux centraux 2c. Il est entendu que ce nombre précis et cette disposition précise des anneaux est donnée à titre d'exemple non limitatif. Le nombre d'anneaux 2 peut varier, leur position également, tout en restant dans le cadre de l'invention.

On se réfère à la figure 4 sur laquelle les anneaux 2a,2b,2c ont été sous numérotés 2a1,2a2,2a3,2a4,2a5,2b1,2b2,2b3,2b4,2c1,2c2 pour décrire leur fonctionnalité, étant entendu que cette figure 4 ne représente qu'une moitié du support 1, Les quatre anneaux périphériques 2a1,2a2 et les deux anneaux de seconde périphérie 2b1 situés vers l'avant de la tête 3 sont destinés à atteindre le lobe frontal. Les six anneaux périphériques 2a2,2a3,2a4 s'étendant depuis le second anneau périphérique avant sont destinés à atteindre le lobe temporal. Les quatre anneaux centraux 2c1,2c2 et les quatre anneaux de seconde périphérie 2b2,2b3 situés au-dessus des oreilles du patient sont destinés à atteindre le lobe pariétal. Les quatre anneaux périphériques 2a4,2a5 les plus en arrière sont destinés à atteindre le cervelet. Et les deux anneaux de seconde périphérie 2b4 situés au-dessus de l'anneau périphérique le plus en arrière, et les deux anneaux périphériques 2a4 à l'avant des anneaux périphériques 2a5 les plus en arrière, sont destinés à atteindre le lobe occipital. Tous les anneaux 2 permettent également d'atteindre plus profondément le thalamus, l'hippocampe et les amygdales.

Les quatre anneaux centraux 2c sont reliés entre eux par des jonctions centrales 4a. Les anneaux de seconde périphérie 2b sont reliés aux anneaux centraux 2c par des jonctions de seconde périphérie 4b. Certains anneaux de seconde périphérie 2b sont également reliés entre eux par des jonctions de seconde périphérie 4b. Les anneaux périphériques 2a sont chacun relié à un anneau de second périphérie 2b par une jonction périphérique 4a. En revanche, les anneaux périphériques 2a ne sont pas reliés entre eux par des jonctions. Il est possible de prévoir que l'une des jonctions 4a,4b,4c comporte une zone plate permettant d'y solidariser une étiquette d'identification du patient.

Selon l'invention, l'absence de liaison entre les anneaux périphériques 2a permet au support 1 de s'adapter à la forme de la tête du patient en s'ouvrant plus ou moins. Restant dans le cadre de l'invention, certains anneaux périphériques 2a pourraient être reliés entre eux tout en conférant cette fonctionnalité d'adaptation. En revanche, si tous les anneaux périphériques 2a sont reliés entre eux, le support 1 ne pourra pas s'adapter à différentes formes de têtes.

Pour ce qui est des anneaux de seconde périphérie 2b, certains sont reliés entre eux pour que le support présente une tenue suffisante pour rester en place sur la tête et maintenir les modules en places dans les anneaux. Le fait que certains anneaux de seconde périphérie 2b ne soient pas reliés entre eux permet également d'entretenir la fonctionnalité d'adaptation du support 1 à toute forme de tête.

La présence ou non et la position des jonctions de seconde périphérie 4b et des jonctions périphériques 4a sont appréciées par l'homme du métier dans un compromis entre la rigidité du support 1 nécessaire pour lui permettre d'être maintenu en position sur la tête du patient et de maintenir le ou les modules, et le caractère d'adaptation du support 1 à toute forme de tête. La présence ou non de ces jonctions peut notamment varier selon le nombre d'anneaux 2 présents sur le support 1 ou encore la rigidité des matériaux employés pour réaliser les anneaux 2 et les jonctions 4.

En tout état de cause, il est essentiel qu'une partie au moins des anneaux périphériques 2a ne soient pas reliés entre eux, et de préférence que tous les anneaux périphériques 2a ne soient pas reliés entre eux. Il apparaît également important pour la rigidité du support qu'au contraire, les anneaux centraux 2c soient reliés entre eux par des jonctions 4a. La liaison intégrale des anneaux centraux 2c dépend du nombre d'anneaux centraux 2c utilisés.

À titre d'exemple, le support de l'invention 1 pourra être réalisé en une seule pièce par moulage de silicone.

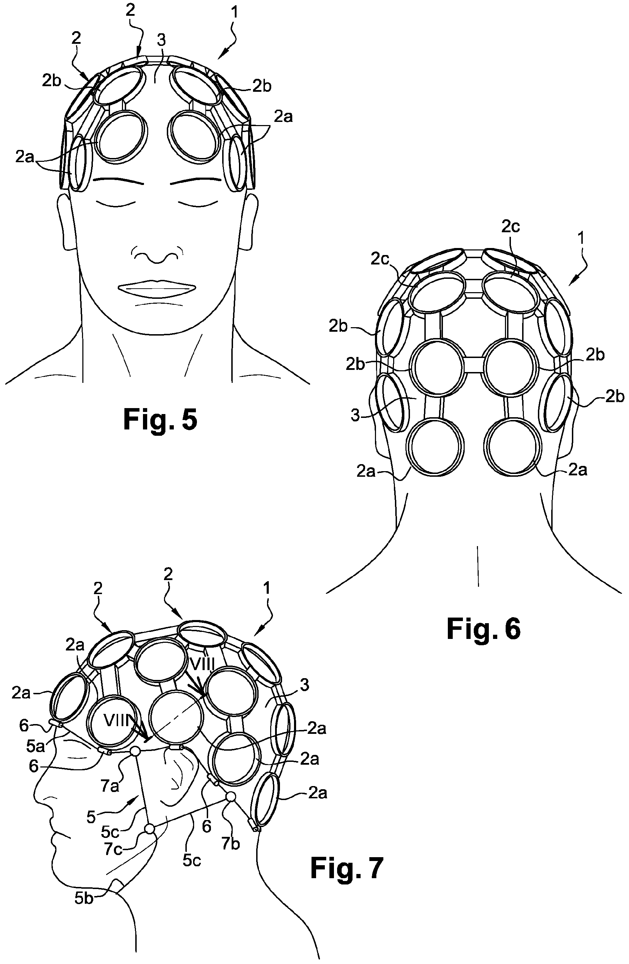

En référence à la figure 7, pour améliorer plus encore le maintien du support 1 sur la tête et pour positionner précisément les anneaux 2 vers les zones du cerveau à traiter, il est prévu une sangle à serrage contrôlé 5. Cette sangle 5 comporte une première partie de liaison 5a qui relie chaque anneau périphérique 2a en passant par un élément tubulaire 6 agencé au niveau du bord inférieur périphérique de chaque anneau périphérique 2a.

La sangle 5 comporte également une mentonnière 5b reliée à la première partie de liaison 5a par quatre éléments de liaison 5c, passant deux à deux de part et d'autre de l'oreille du patient.

On peut prévoir trois points de serrage 7a,7b,7c parmi lesquels deux points de serrage 7a,7b sont situés à la jonction des éléments de liaison 5c et de la première partie de liaison 5a, et le troisième point de serrage est situé à la jonction entre la mentonnière 5b et les deux éléments de liaison 5c.

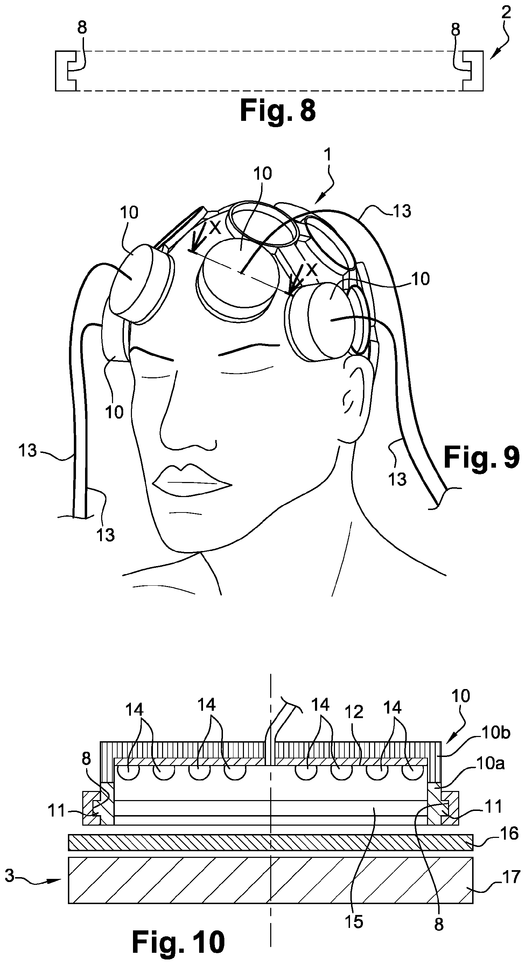

Pour améliorer plus encore la tenue des modules (qui seront décrit plus loin) dans les anneaux 2, on peut prévoir, en référence à la figure 8, la présence d'une rainure 8 au niveau de la face interne de l'anneau 2. Cette rainure 8 est destinée à coïncider avec une nervure circulaire ménagée sur la surface externe du module associé. La présence de la rainure 8 permet non seulement d'améliorer la tenue du module, mais apporte également un positionnement plus précis du module pour que les rayonnements soient précisément dirigés vers les zones ciblées du cerveau.

On se réfère aux figures 9 et 10 pour décrire les modules et l'assemblage des modules sur les anneaux du support de l'invention 1. L'ensemble d'au moins un module et du support 1 formant le dispositif d'irradiation transcutanée, en l'occurrence de type transcrânienne dans cet exemple, de l'invention.

L'assemblage du module 10 dans un anneau 2 est réalisé par insertion coaxiale du module 10 à l'intérieur de l'anneau 2. Le module 10 présente à cet effet une forme générale cylindrique permettant d'assurer le maintien coaxial du module 10 dans l'anneau 2. Cette configuration coaxiale est importante car elle permet d'assurer le positionnement et le maintien fixe du module 10 dans l'axe de l'anneau dédié, ce qui ne peut pas être le cas lorsque le module se présente, comme dans l'art antérieur, sous forme d'une sonde munie d'une poignée et de câbles reliés à dispositif de contrôle et d'alimentation, l'anneau formant alors dans ce cas uniquement la fonction d'indicateur de position.

Comme évoqué précédemment, lorsque les anneaux 2 sont pourvus d'une rainure interne 8, la surface externe du module 10 présente une nervure correspondante circulaire 11 venant se loger dans la rainure 8 pour assurer le positionnement fixe et précis du module 10 dans l'axe de l'anneau 2.

Le module 10 comporte une carte électronique 12 alimentée électriquement via un câble électrique 13 qui s'étend en dehors du module 10. Le câble électrique 13 renferme également des fils de données (par exemple de type bus CAN), un fil d'alimentation de la ou des sources d'irradiation et un ou plusieurs fils de masse. Le module 10 comporte, selon l'invention, la source d'irradiation, ce qui lui permet d'adopter cette géométrie compacte, ici cylindrique. Dans cet exemple, la source d'irradiation est composée de diodes laser et/ou de diodes électroluminescentes (LEDs) 14 émettant dans le spectre visible et/ou dans l'infrarouge proche (longueurs d'onde comprises entre 0,78 et 3 µm selon le découpage de la norme ISO 20473:2007) et/ou dans l'infrarouge IR-A (longueurs d'onde comprises entre 0,7 et 1,4 µm selon le découpage de la Commission internationale de l'éclairage). Le traitement appliqué est par exemple un traitement de photothérapie dont les caractéristiques en termes de durée et d'intensité ne font pas l'objet de la présente invention et sont connues de l'homme du métier.

Le rayonnement électromagnétique généré par les sources lumineuses 14 traverse un guide optique 15 situé en partie inférieure du module 10 à proximité des cheveux 16 et de la peau 17 de la tête 3 du patient. Le rayonnement pénètre alors vers la zone ciblée du cerveau.

Le module 10 peut être réalisé en une seule pièce, ou en deux pièces comme représenté sur la figure 10. Dans ce dernier cas, le module comporte une partie inférieure annulaire 10a, et une partie supérieure annulaire 10b. Les parties inférieures 10a et supérieure 10b peuvent s'assembler par tout moyen connu de l'homme du métier, notamment par un système à baïonnette, par vissage ou par encliquetage.

Comme illustré sur la figure 10, la nervure circulaire 11 est agencée sur la partie inférieure 10a qui comporte le guide optique 15, cette partie inférieure 10a peut ainsi rester en place sur l'anneau 2 entre deux traitements. La partie supérieure 10b comporte la carte électronique et la ou les sources d'irradiation, et peut venir être positionnée sur la partie inférieure au moment du traitement.

## Revendications

1. Support de module d'irradiation transcutanée, comportant des moyens de positionnement (2, 4 ; 5) dudit support (1) au niveau de la zone à irradier, et au moins un anneau (2) réalisé en un matériau élastique et/ou souple et apte à assurer la fixation par emprise élastique d'un module d'irradiation (10), le support de module d'irradiation comportant une pluralité d'anneaux (2) reliés entre eux par des éléments de jonction (4) en formant un support de module d'irradiation transcrânienne (1) apte à être positionné sur la tête (3) d'un utilisateur, le support de module d'irradiation étant **caractérisé en ce que** les anneaux (2) comportent des anneaux périphériques (2a - 2a1, 2a2, 2a3, 2a4, 2a5) dont certains au moins ne sont pas reliés entre eux par des éléments de jonction (4).

2. Support selon la revendication 1, **caractérisé en ce que** les anneaux (2) sont symétriquement disposés de part et d'autre d'un axe (XX') coïncidant avec l'axe médian de la tête (3) lorsque le support (1) est en place sur la tête (3) de l'utilisateur.

3. Support selon la revendication 2, **caractérisé en ce que** les anneaux périphériques (2a) ne sont pas reliés entre eux par des éléments de jonction (4).

4. Support selon l'une quelconque des revendications 2 et 3, **caractérisé en ce que** les anneaux (2) comportent des anneaux de seconde périphérie (2b - 2b1, 2b2, 2b3, 2b4) et quatre anneaux centraux (2c - 2c1, 2c2), **en ce qu'**au moins certains anneaux de seconde périphérie (2b) ne sont pas reliés entre eux par des éléments de jonction (4), et **en ce que** les quatre anneaux centraux (2c) sont reliés entre eux par des éléments de jonction (4c).

5. Support selon l'une quelconque des revendications 2 et 3, **caractérisé en ce qu'**il comporte une sangle à serrage contrôlé (5) comportant au moins une partie de liaison (5a) reliant les anneaux périphériques (2a).

6. Support selon la revendication 4, **caractérisé en ce que** la sangle (5) comporte une mentonnière (5b) se prolongeant de chaque côté du support (1) jusqu'à deux éléments de liaisons (5c) qui sont destinés à être respectivement disposés de chaque côté d'une oreille de l'utilisateur et qui forment jonction entre ladite mentonnière (5b) et ladite partie de liaison (5a).

7. Support selon l'une quelconque des revendications précédentes, **caractérisé en ce que** la face interne de chaque anneau (2) comporte une rainure (8) d'aide à la fixation du module dédié (10).

8. Support selon l'une quelconque des revendications précédentes, caractérisé en ce les anneaux (2) sont réalisés en un matériau élastique souple et/ou élastique.

9. Dispositif d'irradiation transcutanée, **caractérisé en ce qu'**il comporte un support selon l'une quelconque des revendications 1 à 8, et au moins un module d'irradiation (10) comportant au moins une source de rayonnement (14) et qui est apte à être coaxialement fixé sur ledit anneau (2).

10. Dispositif selon la revendication 9, **caractérisé en ce que** la source de rayonnement comporte une série de diodes laser infrarouge, et/ ou de diodes électroluminescentes (LEDs) émettant dans le spectre visible ou infrarouge.

## Patentansprüche

1. Träger für ein Modul zur transkutanen Bestrahlung, umfassend Mittel (2, 4; 5) zur Positionierung des Trägers (1) in Bezug auf die zu bestrahlende Zone und mindestens einen Ring (2), der aus einem elastischen und/oder weichen Material hergestellt ist und dazu geeignet ist, die Fixierung eines Bestrahlungsmoduls (10) durch elastische Einwirkung sicherzustellen, wobei der Bestrahlungsmodulträger eine Vielzahl von Ringen (2) umfasst, die durch Verbindungelemente (4) miteinander verbunden sind, um einen Träger (1) für ein Modul zur transkraniellen Bestrahlung zu bilden, der dazu geeignet ist, auf dem Kopf (3) eines Benutzers positioniert zu werden, wobei der Bestrahlungsmodulträger **dadurch gekennzeichnet ist, dass** die Ringe (2) Randringe (2a - 2a1, 2a2, 2a3, 2a4, 2a5) umfassen, von denen mindestens einige nicht durch Verbindungselemente (4) miteinander verbunden sind.

2. Träger nach Anspruch 1, **dadurch gekennzeichnet, dass** die Ringe (2) symmetrisch auf beiden Seiten einer Achse (XX') angeordnet sind, die mit der Mittelachse des Kopfs (3) zusammenfällt, wenn der Träger (1) auf dem Kopf (3) des Benutzers vorliegt.

3. Träger nach Anspruch 2, **dadurch gekennzeichnet, dass** die Randringe (2a) nicht durch Verbindungselemente (4) miteinander verbunden sind.

4. Träger nach einem der Ansprüche 2 und 3, **dadurch gekennzeichnet, dass** die Ringe (2) zweite Randringe (2b - 2b1, 2b2, 2b3, 2b4) und vier zentrale Ringe (2c - 2c1, 2c2) umfassen, dass mindestens einige zweite Randringe (2b) nicht durch Verbindungselemente (4) miteinander verbunden sind und dass die vier zentralen Ringe (2c) durch Verbindungselemente (4c) miteinander verbunden sind.

5. Träger nach einem der Ansprüche 2 und 3, **dadurch gekennzeichnet, dass** er ein Band (5) zum kontrollierten Festziehen umfasst, das mindestens einen Bindungsteil (5a) umfasst, der die Randringe (2a) verbindet.

6. Träger nach Anspruch 4, **dadurch gekennzeichnet, dass** das Band (5) einen Kinnriemen (5b) umfasst, der sich auf jeder Seite des Trägers (1) bis zu zwei Verbindungselementen (5c) ausdehnt, die dazu vorgesehen sind, jeweils auf jeder Seite eines Ohrs des Benutzers angeordnet zu werden, und die eine Verbindung zwischen dem Kinnriemen (5b) und dem Bindungsteil (5a) bilden.

7. Träger nach einem der vorhergehenden Ansprüche, **dadurch gekennzeichnet, dass** die Innenfläche jedes Rings (2) eine Nut (8) zur Unterstützung der Fixierung des spezifischen Moduls (10) umfasst.

8. Träger nach einem der vorhergehenden Ansprüche, **dadurch gekennzeichnet, dass** die Ringe (2) aus einem elastischen, weichen und/oder elastischen Material hergestellt sind.

9. Vorrichtung zur transkutanen Bestrahlung, **dadurch gekennzeichnet, dass** sie einen Träger nach einem der Ansprüche 1 bis 8 und mindestens ein Bestrahlungsmodul (10) umfasst, das mindestens eine Strahlungsquelle (14) umfasst und das dazu geeignet ist, koaxial an dem Ring (2) fixiert zu werden.

10. Vorrichtung nach Anspruch 9, **dadurch gekennzeichnet, dass** die Strahlungsquelle eine Reihe von Infrarotlaserdioden und/oder Elektrolumineszenzdioden (LED) umfasst, die im sichtbaren oder Infrarotspektrum abstrahlen.

## Claims

1. Support for a transcutaneous irradiation module, comprising means for positioning (2, 4; 5) said support (1) on the zone to be irradiated, and at least one ring (2) made from an elastic and/or flexible material and able to provide the fastening via elastic grip of an irradiation module (10), with the support for an irradiation module comprising a plurality of rings (2) connected together by junction elements (4) by forming a support for a transcranial irradiation module (1) able to be positioned on the head (3) of a user, the support for an irradiation module being **characterised in that** the rings (2) comprise peripheral rings (2a - 2a1, 2a2, 2a3, 2a4, 2a5) of which some at least are not connected together by junction elements (4).

2. Support according to claim 1, **characterised in that** the rings (2) are symmetrically arranged on either side of an axis (XX') coinciding with the median axis of the head (3) when the support (1) is in place on the head (3) of the user.

3. Support according to claim 2, **characterised in that** the peripheral rings (2a) are not connected together by junction elements (4).

4. Support according to any of claims 2 and 3, **characterised in that** the rings (2) comprise second periphery rings (2b - 2b1, 2b2, 2b3, 2b4) and four central rings (2c - 2c1, 2c2), **in that** at least some second periphery rings (2b) are not connected together by junction elements (4), and **in that** the four central rings (2c) are connected together by junction elements (4c) .

5. Support according to any of claims 2 and 3, **characterised in that** it comprises a controlled tightening strap (5) that comprises at least one connection portion (5a) connecting the peripheral rings (2a) .

6. Support according to claim 4, **characterised in that** the strap (5) comprises a chinstrap (5b) extending on each side of the support (1) to two connection elements (5c) which are intended to be respectively arranged on each side of an ear of the user and which form a junction between said chinstrap (5b) and said connection portion (5a).

7. Support according to any preceding claim, **characterised in that** the internal face of each ring (2) comprises a groove (8) for assisting in the fastening of the dedicated module (10).

8. Support according to any preceding claim, **characterised in that** the rings (2) are made from a flexible elastic and/or elastic material.

9. Transcutaneous irradiation device, **characterised in that** it comprises a support according to any of claims 1 to 8, and at least one irradiation module (10) comprising at least one source of radiation (14) and which is able to be coaxially fastened onto said ring (2).

10. Device according to claim 9, **characterised in that** the source of radiation comprises a series of infrared laser diodes, and/ or light-emitting diodes (LEDs) emitting in the visible or infrared spectrum.
